# EUROPEAN PATENT APPLICATION

(11) **EP 2 614 816 A1**
(43) Date of publication of application: **17.07.2013**
(21) Application number: 11821366.9
(22) Date of filing: 08.04.2011
(51) Int. Cl.: A61K 9/16, A61K 9/26, A61K 31/335, A61K 47/10, A61K 47/26, A61K 47/32, A61P 37/08

(54) **GRANULES CONTAINING BITTER DRUG AND ORALLY DISINTEGRATING TABLET**

(30) Priority: 31.08.2010 JP 2010194755
(71) Applicant: Kyowa Hakko Kirin Co., Ltd., Tokyo 100-8185 (JP)
(72) Inventor: MIYAMOTO, Yuji, Tokyo 100-8155 (JP); TOKUDA, Takuya, Tokyo 100-8185 (JP); OTA, Motohiro, Tokyo 100-8185 (JP); ISHIKAWA, Yasuhiro, Tokyo 100-8185 (JP)
(74) Representative: Vossius & Partner
(86) International application number: PCT/JP2011/058919
(87) International publication number: WO 2012/029348

(57) **Abstract**

It is an object of the present invention to provide a powder, a granule, an orally-disintegrating tablet, and the like that contain a drug causing bitterness, and that can suppress the bitterness in the mouth and improve solubility thereof in the stomach. The present invention provides a drug-containing granule comprising (a) a core particle that contains a drug causing bitterness, and (b) a masking coating that coats the core particle,
wherein the masking coating contains:
at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer; and
at least one diluent selected from D-mannitol, lactose, trehalose, xylitol, maltitol, and erythritol,
an orally-disintegrating tablet comprising the drug-containing granule, and the like.

## Description

### [Technical Field]

The present invention relates to granules and orally-disintegrating tablets containing a drug causing bitterness; processes for producing the same; and the like.

### [Background Art]

Taking oral solid medical drug products such as usual tablets and capsules can be problematic for the elderly having a weakened ability to swallow. The same problem is presented also for children with a low swallowing ability. Powders and granules are among the dosage forms suited for the elderly and children. There is also a recent demand for the development of orally-disintegrating tablets that quickly dissolve or disintegrate in the mouth.
Powders, granules, and orally-disintegrating tablets, when containing a drug causing bitterness, can be difficult to be taken as the drug quickly dissolves in the mouth and causes bitterness. In order to suppress dissolving of the drug and prevent the bitterness, the amount of the drug dissolving in the mouth needs to be very small, because the bitterness in general is easily perceived even in small amounts. However, efforts made to the powders, granules, and orally-disintegrating tablets to suppress dissolving of the drug suppress the amount of dissolution thereof not only in the mouth but in the stomach and small intestine. This may have adverse effects on the therapeutic effect of the drug.
Attempts are made to suppress bitterness in the mouth (suppress an amount of dissolution thereof in the mouth) and improve solubility thereof in the stomach for powders, granules, and orally-disintegrating tablets that contain a drug causing bitterness, as described in Patent Documents 1 to 3, and the like. Patent Documents 1 and 2 describe coating a drug with a film base such as an enteric film base to reduce an uncomfortable taste of a drug, and using a glidant and a disintegrant for the enteric film base.
Patent Document 3 describes a principal agent particle obtained by coating a basic or acidic principal drug particle with a water-insoluble coating film that contains an acidic substance and a basic substance for the basic principal agent and the acidic principal agent, respectively, and an orally-disintegrating tablet using the same. According to Patent Document 3, the coating agent may also contain excipients known in the art, such as plasticizers, sweeteners, dispersion stabilizers, diluents, and lubricants, as required.

### [Citation List]

### [Patent Documents]

[Patent Document 1] Japanese Published Unexamined Patent Application No. 2007-063263
[Patent Document 2] Japanese Published Unexamined Patent Application No. 2008-214334
[Patent Document 3] Japanese Published Unexamined Patent Application No. 2008-260712

### [Summary of the Invention]

### [Problems to be Solved by the Invention]

It is an object of the present invention to provide a powder, a granule, an orally-disintegrating tablet, and the like that contain a drug causing bitterness, and that can suppress the bitterness in the mouth (suppress an amount of dissolution thereof in the mouth) and improve solubility thereof in the stomach.

### [Means for Solving the Problems]

The present invention relates to the following (1) to (11).
(1) A drug-containing granule comprising (a) a core particle that contains a drug causing bitterness, and (b) a masking coating that coats the core particle,
   wherein the masking coating contains:
   at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer in 20 to 70 weight% of the coating; and
   at least one diluent selected from D-mannitol, lactose, trehalose, xylitol, maltitol, and erythritol in 40 to 250 weight% of the polymer.
(2) A drug-containing granule comprising (a) a core particle that contains cinacalcet, topiramate, olopatadine, or a pharmaceutically acceptable salt thereof as a drug causing bitterness, and (b) a masking coating that coats the core particle,
   wherein the masking coating contains methacrylic acid-ethyl acrylate copolymer, and at least one diluent selected from D-mannitol, lactose, trehalose, xylitol, maltitol, and erythritol.
(3) The drug-containing granule according to the above (1) or (2), wherein the core particle contains at least one diluent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.
(4) The drug-containing granule according to any one of the above (1) to (3), wherein the drug-containing granule has a weight average particle diameter of 100 to 350 µm.
(5) An orally-disintegrating tablet comprising the drug-containing granule of any one of the above (1) to (4), and a powder for an orally-disintegrating tablet that does not contain the drug causing bitterness.
(6) A process for producing a drug-containing granule that comprises (a) a core particle that contains a drug causing bitterness, and (b) a masking coating that coats the core particle,
   the process comprising the steps of:
   producing the core particle; and
   forming a masking coating by coating the core particle with a coating liquid that contains:
      at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer in 20 to 70 weight% of the masking coating; and
      at least one diluent selected from D-mannitol, lactose, trehalose, xylitol, maltitol, and erythritol in 40 to 250 weight% of the polymer.
(7) The process according to the above (6), wherein the step of producing the core particle that contains a drug causing bitterness is the step of granulating the drug causing bitterness and at least one diluent selected from D-mannitol, lactose, trehalose, xylitol, maltitol, and erythritol with a binder liquid that contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.
(8) A process for producing a drug-containing granule that comprises (a) a core particle that contains cinacalcet, topiramate, olopatadine, or a pharmaceutically acceptable salt thereof as a drug causing bitterness, and (b) a masking coating that coats the core particle,
   the process comprising the steps of:
   producing the core particle; and
   forming a masking coating by coating the core particle with a coating liquid that contains methacrylic acid-ethyl acrylate copolymer, and at least one diluent selected from D-mannitol, lactose, trehalose, xylitol, maltitol, and erythritol.
(9) The process according to the above (8), wherein the step of producing the core particle is the step of granulating cinacalcet, topiramate, olopatadine, or a pharmaceutically acceptable salt thereof, and at least one diluent selected from D-mannitol, lactose, trehalose, xylitol, maltitol, and erythritol with a binder liquid that contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.
(10) The process according to any one of the above (6) to (9), wherein the core particle is made to have a weight average particle diameter of 75 to 150 µm.
(11) A process for producing an orally-disintegrating tablet,
   the process comprising the steps of:
   mixing the drug-containing granule obtained by the process of any one of the above (6) to (10) with a powder for an orally-disintegrating tablet that does not contain the drug causing bitterness; and
   compressing the mixed powder.

### [Effect of the Invention]

The present invention can provide a powder, a granule, an orally-disintegrating tablet, and the like that contain a drug causing bitterness, and that can suppress the bitterness in the mouth (suppress an amount of dissolution thereof in the mouth) and improve solubility thereof in the stomach.

### [Brief Description of the Drawings]

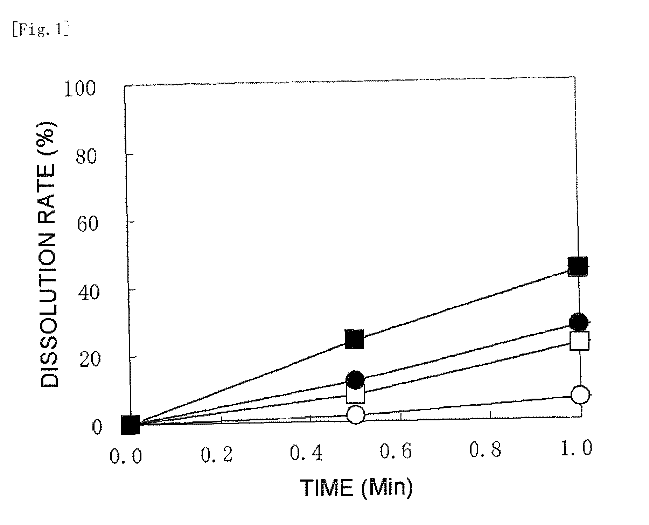

[Fig. 1] Fig. 1 shows the results of dissolution tests performed for olopatadine hydrochloride-containing granules obtained in Examples 1 to 3 and Comparative Example 1; the horizontal axis, sampling time; the vertical axis, dissolution rate; open square, Example 1; solid circle, Example 2; solid square, Example 3; open circle, Comparative Example 1.

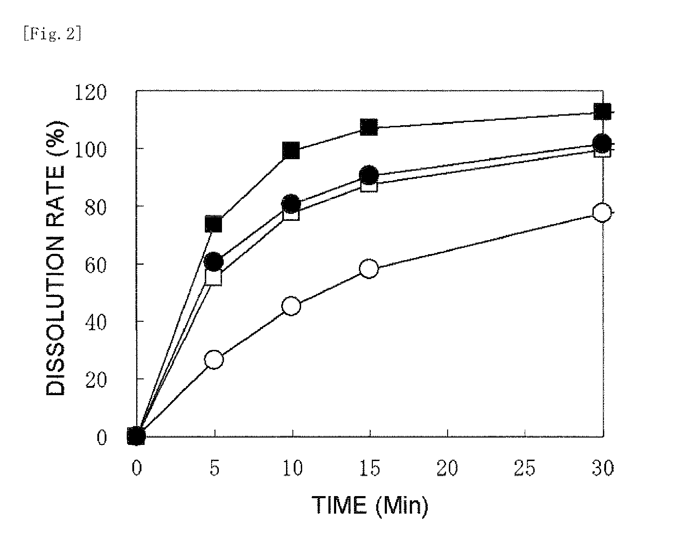

[Fig. 2] Fig. 2 shows the results of dissolution tests performed for olopatadine hydrochloride-containing granules obtained in Examples 1 to 3 and Comparative Example 1; the horizontal axis, sampling time; the vertical axis, dissolution rate; open square, Example 1; solid circle, Example 2; solid square, Example 3; open circle, Comparative Example 1.

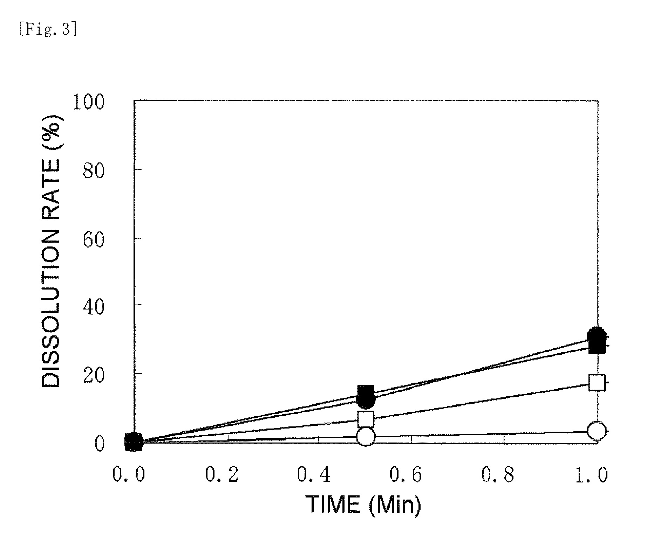

[Fig. 3] Fig. 3 shows the results of dissolution tests performed for olopatadine hydrochloride-containing granules obtained in Examples 4 to 6 and Comparative Example 2; the horizontal axis, sampling time; the vertical axis, dissolution rate; open square, Example 4; solid circle, Example 5; solid square, Example 6; open circle, Comparative Example 2.

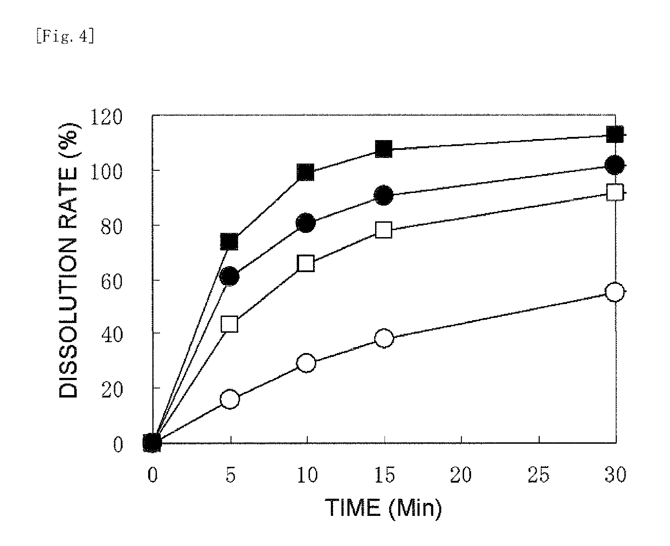

[Fig. 4] Fig. 4 shows the results of dissolution tests performed for olopatadine hydrochloride-containing granules obtained in Examples 4 to 6 and Comparative Example 2; the horizontal axis, sampling time; the vertical axis, dissolution rate; open square, Example 4; solid circle, Example 5; solid square, Example 6; open circle, Comparative Example 2.

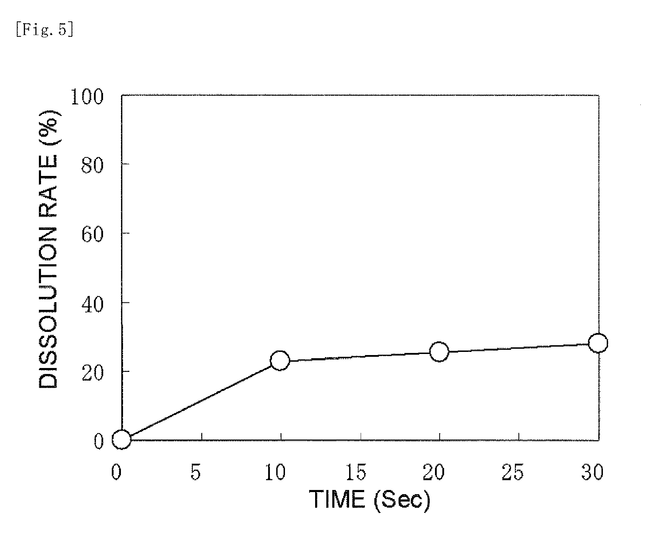

[Fig. 5] Fig. 5 shows the result of a dissolution test performed for olopatadine hydrochloride-containing orally-disintegrating tablets obtained in Example 8; the horizontal axis, sampling time; the vertical axis, dissolution rate.

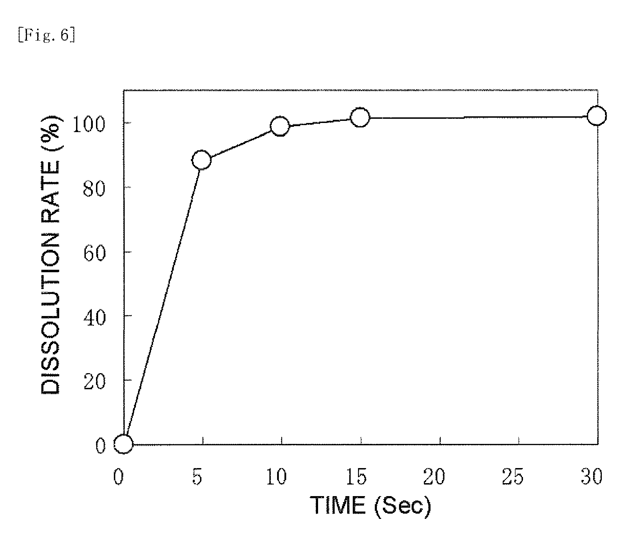

[Fig. 6] Fig. 6 shows the result of a dissolution test performed for olopatadine hydrochloride-containing orally-disintegrating tablets obtained in Example 8; the horizontal axis, sampling time; the vertical axis, dissolution rate.

### [Mode for Carrying Out the Invention]

The drug-containing granule of the present invention comprises (a) a core particle that contains a drug causing bitterness, and (b) a masking coating that coats the core particle.

The drug causing bitterness used in the present invention is not particularly limited, as long as it causes bitterness.
Preferred examples are drugs that require its dissolution amount in the mouth to be 40% or less, more preferably 30% or less, further preferably 25% or less of a single dose to suppress the bitterness in the mouth. The amount of drug dissolution in the mouth means the amount of drug that dissolves in the mouth during the residence time, for example, 30 seconds, of the powder, granule, orally-disintegrating tablet, and the like in the mouth after being ingested. For example, the amount of drug dissolution in the mouth means that the drug dissolves in 40% or less, more preferably 30% or less, further preferably 25% or less of a single dose within 30 seconds in a test performed according to method 2 of the dissolution test (rotation paddle method) described in Japanese Pharmacopoeia 15 (JP) under 37°C, 50 rpm conditions with 900 mL of water used as a test liquid, or a test performed according to the syringe barrel inversion method (an in vitro test that simulates an amount of drug dissolution in the mouth; a single tablet is placed in a syringe barrel, and the barrel is repeatedly inverted once in every 10 seconds after adding 5 mL of water thereto; see Yasuhiko Nakamura, Research on Masking Technique for Uncomfortable Taste and Sustained-Release Preparation, Phaem Tech Japan, 2005, Vol. 21, No. 5, p. 163-169).
Note that the term "bitterness" as used herein is inclusive of tastes such as acid taste, salty taste, bitter taste, pungency, astringency, acrid taste, alkaline taste, and metallic taste, and encompasses tastes and stimulations that present an obstacle to drug ingestion.
Specific examples of the drug causing bitterness used in the present invention include cinacalcet, topiramate, olopatadine, pharmaceutically acceptable salts thereof, and the like.

The pharmaceutically acceptable salts are inclusive of, for example, pharmaceutically acceptable acid addition salts, metal salts, ammonium salts, organic amine addition salts, amino acid addition salts, and the like. Examples of the pharmaceutically acceptable acid addition salts include inorganic salts (for example, such as hydrochloride, hydrobromate, nitrate, sulfate, and phosphate), and organic acid salts (for example, such as acetate, maleate, fumarate, tartrate, and citrate). Examples of the pharmaceutically acceptable metal salts include alkali metal salts (for example, such as lithium salts, sodium salts, and potassium salts), alkaline-earth metal salts (for example, such as magnesium salts, and calcium salts), aluminum salts, and zinc salts. Examples of the pharmaceutically acceptable ammonium salts include, for example, salts of ammonium, tetramethylammonium, and the like. Examples of the pharmaceutically acceptable organic amine addition salts include, for example, addition salts of morpholine, piperidine, and the like. Examples of the pharmaceutically acceptable amino acid addition salts include, for example, addition salts of lysine, glycine, phenylalanine, aspartic acid, glutamic acid, and the like.

In the core particle that contains a drug causing bitterness, the drug causing bitterness is contained in preferably 0. 5 to 80 weight%, more preferably 1 to 50 weight%, further preferably 2 to 30 weight%, most preferably 5 to 20 weight% of the core particle. The drug causing bitterness has a volume average particle diameter of preferably 2 to 150 µm, more preferably 15 to 100 µm, further preferably 25 to 50 µm. In the present invention, a volume average particle diameter may be determined by calculation, for example, by measuring unidirectional particle diameters by microscopy or by using a laser method and regarding the measured values as spherical particle diameters.

The core particle that contains a drug causing bitterness may contain common excipients, in addition to the drug causing bitterness. Preferably, the core particle contains at least one diluent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer. More preferably, the core particle contains at least one diluent selected from D-mannitol, lactose, and erythritol, and at least one polymer selected from ethyl acrylate-methyl methacrylate copolymer, methacrylic acid-ethyl acrylate copolymer, and ethyl acrylate-methyl methacrylate-ethyl ammonium trimethyl chloride methacrylate copolymer. Most preferably, the core particle contains D-mannitol and methacrylic acid-ethyl acrylate copolymer. In the present invention, the core particle that contains a drug causing bitterness preferably contains at least one diluent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, because it can more desirably suppress the bitterness in the mouth (suppress an amount of dissolution thereof in the mouth) and improve solubility thereof in the stomach.

The core particle that contains a drug causing bitterness contains at least one diluent selected from D-mannitol, lactose, trehalose, xylitol, maltitol, and erythritol, preferably at least one diluent selected from D-mannitol, lactose, and erythritol, more preferably D-mannitol in 20 to 95 weight%, more preferably 40 to 90 weight%, further preferably 60 to 85 weight%.
The core particle that contains a drug causing bitterness contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, preferably at least one polymer selected from ethyl acrylate-methyl methacrylate copolymer, methacrylic acid-ethyl acrylate copolymer, and ethyl acrylate-methyl methacrylate-ethyl ammonium trimethyl chloride methacrylate copolymer, more preferably methacrylic acid-ethyl acrylate copolymer in 0.01 to 50 weight%, more preferably 0.1 to 20 weight%, further preferably 1 to 10 weight%.

The core particle that contains a drug causing bitterness may contain other drugs and/or other excipients, in addition to the drug causing bitterness, at least one diluent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.
Examples of other excipients include those used for, for example, diluents, disintegrants, binders, lubricants, and the like in common solid preparations.
Examples of the diluents added as other excipients include sugars (for example, sucrose, maltose, and the like), sugar alcohols (for example, sorbitol, and the like), starches (for example, corn starch, rice starch, wheat starch, and the like), celluloses (for example, microcrystalline cellulose, powder cellulose, and the like), poorly water-soluble inorganic salts (for example, talc, light anhydrous silicic acid, and the like), and the like. These may be used either alone or in combinations of two or more.
Examples of the binders added as other excipients include cellulose derivatives (for example, methyl cellulose, carmellose, carboxypropylcellulose, hydroxypropylcellulose, hydroxypropyl methylcellulose, and the like), celluloses (for example, microcrystalline cellulose, and the like), starches (for example, pregelatinated starch, and the like), polyvinyl alcohols, polyvinyl pyrrolidones, pullulans, dextrins, gum arabic, gelatins, and the like. These may be used either alone or in combinations of two or more.
Examples of the lubricants added as other excipients include magnesium stearate, calcium stearate, hardened oil, sucrose fatty acid ester, polyethylene glycol, and the like. These may be used either alone or in combinations of two or more.

Examples of the disintegrants added as other excipients include cellulose derivatives (for example, crosscarmellose sodium, carmellose sodium, carmellose potassium, carmellose calcium, low-substituted hydroxypropylcellulose, and the like), starches (for example, pregelatinated starch, partially pregelatinated starch, and the like), starch derivatives (carboxymethyl starch sodium, hydroxypropyl starch, and the like), crospovidone, bentonite, and the like. These may be used either alone or in combinations of two or more. For desirable productivity and/or quick drug solubility, the core particle that contains a drug causing bitterness according to the present invention preferably contains at least one disintegrant selected from crosscarmellose sodium, low-substituted hydroxypropylcellulose, hydroxypropyl starch, crospovidone, and the like. Further, for improved stability, the core particle that contains topiramate and a pharmaceutically acceptable salt thereof preferably contains crosscarmellose sodium or carmellose sodium, and, more preferably, the core particle that contains olopatadine and a pharmaceutically acceptable salt thereof contains low-substituted hydroxypropylcellulose.
The core particle that contains a drug causing bitterness contains a disintegrant, preferably at least one disintegrant selected from crosscarmellose sodium, low-substituted hydroxypropylcellulose, hydroxypropyl starch, and crospovidone in 0.5 to 50 weight%, more preferably 1 to 25 weight%, further preferably 3 to 15 weight%.
Further, the core particle that contains a drug causing bitterness may contain a dye, a light-shielding agent, a flavoring ingredient, and the like. Examples of the dye, the light-shielding agent, or the flavoring ingredient include titanium oxides, iron oxides (specifically, yellow ferric oxide, ferric oxide, yellow ferrous oxide, black iron oxide, and the like), zinc oxides, silicon oxides, red iron oxide, carbon blacks, medicinal carbon, barium sulfate, food yellow 4 aluminum lake, food red 2, food red 3, food red 102, copper chlorophin, various flavoring ingredients, and the like.

The core particle that contains a drug causing bitterness may have any shape, including a sphere, a columnar shape, an irregular shape, and the like. The core particle of the present invention can be desirably masked even when it is an irregular particle obtained by, for example, fluidized bed granulation. The core particle has a weight average particle diameter of preferably 30 to 500 µm, more preferably 50 to 300 µm, further preferably 75 to 150 µm. In the present invention, weight average particle diameter may be determined, for example, by sieving.

The masking coating contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, preferably at least one polymer selected from ethyl acrylate-methyl methacrylate copolymer, methacrylic acid-ethyl acrylate copolymer, and ethyl acrylate-methyl methacrylate-ethyl ammonium trimethyl chloride methacrylate copolymer, more preferably methacrylic acid-ethyl acrylate copolymer. In the present invention, the masking coating preferably contains methacrylic acid-ethyl acrylate copolymer, because it can also further improve stability.

In the present invention, examples of the methacrylic acid copolymer S and the methacrylic acid copolymer L include those described in, for example, JP or Japanese Pharmaceutical Excipients (JPE).
As used herein, the methacrylic acid-ethyl acrylate copolymer is a copolymer resin of methacrylic acid and ethyl acrylate. Examples include copolymer resins of methacrylic acid and ethyl acrylate from among the JPE dry methacrylic acid copolymer LD (Eudragit L100-55; Roehm Pharma Gmbh, and the like), the JPE methacrylic acid copolymer LD (Eudragit L30D-55; Roehm Pharma Gmbh, and the like), and the like.
In the present invention, the ethyl acrylate-methyl methacrylate copolymer is a copolymer resin of ethyl acrylate and methyl methacrylate. Examples include copolymers of ethyl acrylate and methyl methacrylate from among the JPE ethyl acrylate-methyl methacrylate copolymer dispersion (Eudragit NE30D; Roehm Pharma Gmbh, and the like), and the like.
In the present invention, the ethyl acrylate-methyl methacrylate-ethyl ammonium trimethyl chloride methacrylate copolymer is a copolymer resin of ethyl acrylate, methyl methacrylate, and ethyl ammonium trimethyl chloride methacrylate. Examples include copolymer resins of ethyl acrylate, methyl methacrylate, and ethyl ammonium trimethyl chloride methacrylate from among the JPE aminoacrylmethacrylate copolymer RS (Eudragit RS100 (Roehm Pharma Gmbh), Eudragit RSPO (Roehm Pharma Gmbh), Eudragit RL100 (Roehm Pharma Gmbh), Eudragit RLPO (Roehm Pharma Gmbh), and the like), and the aminoacrylmethacrylate copolymer RS dispersion (Eudragit RL30D (Roehm Pharma Gmbh), Eudragit RS30D (Roehm Pharma Gmbh), and the like), and the like.

The masking coating contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, preferably at least one polymer selected from ethyl acrylate-methyl methacrylate copolymer, methacrylic acid-ethyl acrylate copolymer, and ethyl acrylate-methyl methacrylate-ethyl ammonium trimethyl chloride methacrylate copolymer, more preferably methacrylic acid-ethyl acrylate copolymer in preferably 20 to 70 weight%, more preferably 30 to 60 weight%, further preferably 40 to 50 weight% of the coating.

Further, the masking coating contains at least one diluent selected from D-mannitol, lactose, trehalose, xylitol, maltitol, and erythritol, preferably at least one diluent selected from D-mannitol and erythritol, more preferably D-mannitol. In the present invention, the masking coating more preferably contains D-mannitol, because it also further improves stability.

In the present invention, examples of D-mannitol, lactose, trehalose, xylitol, maltitol, and erythritol include those described in, for example JP or JPE. These are inclusive of hydrates (for example, lactose hydrate, and the like).

The masking coating contains at least one diluent selected from D-mannitol, lactose, trehalose, xylitol, maltitol, and erythritol in preferably 40 to 250 weight%, more preferably 60 to 150 weight%, further preferably 80 to 120 weight% with respect to the total amount of the polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.
Further, the total amount of at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, and at least one diluent selected from D-mannitol, lactose, trehalose, xylitol, maltitol, and erythritol is preferably 50 to 99 weight%, more preferably 70 to 98 weight%, further preferably 80 to 95 weight% of the masking coating.

The masking coating may contain a compound having light-shielding performance. Examples of such compounds include light-shielding metallic compounds, silicon compounds, organic compounds, complex substances, and the like. Preferred examples include titanium oxides, iron oxides (specifically, yellow ferric oxide, ferric oxide, yellow ferrous oxide, black iron oxide, and the like), zinc oxides, silicon oxides, red iron oxide, carbon blacks, medicinal carbon, barium sulfate, food yellow 4 aluminum lake, food red 2, food red 3, food red 102, copper chlorophin, and the like.
The masking coating may also contain, for example, other coating bases, plasticizers, and other coating agents, and the like.
Examples of other coating bases include a stomach-soluble film coating base, an enteric film coating base, a sustained-release film coating base, and the like. Examples of the stomach-soluble film coating base include synthetic high polymers such as polyvinyl acetal diethylaminoacetate, and the like. Examples of the enteric film coating base include natural materials such as hydroxypropyl methylcellulose phthalate, hydroxypropyl methylcellulose acetate succinate, carboxymethylethylcellulose, shellac, and the like. Examples of the sustained-release film coating base include cellulose polymers such as ethylcellulose, and the like, preferably ethylcellulose.

Examples of the plasticizers include esters such as triethyl citrate, middle-chain triglyceride, diethyl phthalate, dibutyl phthalate, triacetin, butyl phthalyl butyl glycolate, glyceryl caprylate; alcohols such as glycerine, propylene glycol, and polyethylene glycol, and the like.

Examples of other coating agents include talc, calcium carbonate, carnauba wax, and the like.

When the masking coating further contains a compound having light-shielding performance, other coating bases, plasticizers, and other coating agents and the like, these may be contained in commonly used amounts, preferably in as small amounts as possible. Note that, for example, ethylcellulose may be contained in an amount that corresponds to the polymer-ethylcellulose weight ratio of 1:4 to 1:0, preferably 1:3 to 3:1, more preferably 2:3 to 3:2, with regard to the at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.

The masking coating is not limited to a single layer, and may be formed from a plurality of layers. In this case, the type and/or the content of the component used for each layer may be varied.

The amount of masking coating needs to be sufficient to form a thickness that can achieve taste masking for the core particle, and may be appropriately set according to the particle diameter of the granule. For example, when the core particle has a weight average particle diameter of 75 to 150 µm, the amount of masking coating is preferably 15 to 120 weight%, more preferably 20 to 90 weight%, further preferably 30 to 70 weight% of the core particle. It is also preferable that the amount of masking coating is the amount that can achieve a quick release in the stomach. As used herein, a quick release in the stomach means that the amount of the drug that dissolves, for example, in a dissolution test performed under 37°C, 50 rpm conditions with 900 mL of test liquid water according to method 2 of the JP dissolution test (rotation paddle method) is preferably 75 weight% or more within 30 minutes of the test, more preferably 75 weight% or more within 15.

The drug-containing granule of the present invention may have any shape, including a sphere, a columnar shape, an irregular shape, and the like. The drug-containing granule of the present invention may be any of what is called a powder, a fine granule (for example, 10 weight% or less of the total amount do not pass through a # 30 (500 µm) sieve), or a granule, as decided depending on the size. The drug-containing granule of the present invention has a weight average particle diameter of preferably 75 to 850 µm, more preferably 100 to 500 µm, further preferably 125 to 350 µm as measured by sieving.

The drug-containing granule of the present invention may be produced by a process that includes, for example, the step of producing a core particle that contains a drug causing bitterness, and the step of forming a masking coating by coating the resulting core particle with a coating liquid that contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, and at least one diluent selected from D-mannitol, lactose, trehalose, xylitol, maltitol, and erythritol.

The core particle that contains a drug causing bitterness may be produced by using methods, for example, such as a wet granulation method, and a dry granulation method. Examples of the wet granulation method include an extrusion granulation method (using a screw extrusion granulation device, a roller extrusion granulation device, and the like), a tumbling granulation method (using a rotary drum granulation device, a centrifugal tumbling granulation device, and the like), a fluidized bed granulation method (using a fluidized bed granulation and drying device, a tumbling fluidized bed granulation device, a stirring fluidized bed granulator, and the like), a stirring granulation method (using a stirring granulation device, and the like), and the like. In any case, it is preferable to, for example, mix the drug and excipients, add a solvent or a binder liquid to the resulting mixture and perform granulation, and dry the resulting granulated material. Examples of the solvent include organic solvents such as ethanol, isopropyl alcohol, and acetone, water, mixed solvents thereof, and the like. Examples of the binder liquid include solutions and dispersions prepared by dissolving or dispersing at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, or a binder (having the same definition as the binders added as other excipients) in organic solvents, for example, such as ethanol, isopropyl alcohol, acetone, and the like, water, mixed solvents thereof, and the like.
Preferred are one or more liquids selected from (a) organic solvent liquids dissolving at least one polymer selected from methacrylic acid copolymer S, methacrylic acid copolymer L, and ethyl acrylate-methyl methacrylate copolymer, (b) liquids obtained by suspending at least one polymer selected from methacrylic acid copolymer S, methacrylic acid copolymer L, and ethyl acrylate-methyl methacrylate-ethyl ammonium trimethyl chloride methacrylate copolymer in an aqueous solution dissolving 30 to 100 weight% of triethyl citrate with respect to the polymer, (c) liquids obtained by suspending JPE dry methacrylic acid copolymer LD and/or JPE aminoacrylmethacrylate copolymer RS in water, and (d) JPE methacrylic acid copolymer LD and/or JPE ethyl acrylate-methyl methacrylate copolymer dispersion.
More preferred are one or more liquids selected from (c) liquids obtained by suspending JPE dry methacrylic acid copolymer LD and/or JPE aminoacrylmethacrylate copolymer RS in water, and (d) JPE methacrylic acid copolymer LD and/or JPE ethyl acrylate-methyl methacrylate copolymer dispersion.
Further preferred are liquids obtained by suspending JPE dry methacrylic acid copolymer LD in water, and JPE methacrylic acid copolymer LD.
The dry granulation method may be, for example, a disintegration granulation method in which flakes are produced with a commercially available dry granulator, or slugs are produced with a tableting machine, and in which the flakes or slugs are crushed with a commercially available crusher or pulverizer to obtain granulated materials, and the like.
More preferably, the core particle is produced by using a stirring granulation method, a tumbling granulation method, or a fluidized bed granulation method, further preferably by using a stirring granulation method.
Preferably, the granulated materials may be appropriately pulverized and/or sieved to have a desired weight average particle diameter.

The drug may have a volume average particle diameter of 2 to 150 µm, for example, by classifying crude crystals with a sieve into volume average particle diameters of 2 to 150 µm. Preferably, the crude crystals are pulverized with a pulverizer categorized into, for example, a high-speed rotation mill, a roller mill, a jet mill, a ball mill, and the like, more preferably with a pulverizer categorized into a high-speed rotation mill to obtain a volume average particle diameter of 15 to 100 µm. Examples of the pulverizers categorized into high-speed rotation mills include hammer mills, pin mills, cage mills, shear mills, impact mills, aerofall mills, and the like, of which hammer mills are preferable.

The process for producing the core particle that contains a drug causing bitterness preferably includes the step of mixing the drug causing bitterness with at least one diluent selected from D-mannitol, lactose, trehalose, xylitol, maltitol, and erythritol.

Further, the process for producing the core particle that contains a drug causing bitterness preferably includes the step of mixing the drug causing bitterness and at least one diluent selected from D-mannitol, lactose, trehalose, xylitol, maltitol, and erythritol with other drugs and/or other excipients (having the same definitions as described above).
In these mixing steps, the mixing machine used is not limited, and the materials may be mixed, for example, with machines dedicated to mixing, such as a V-shaped mixing machine, a cone-shaped mixing machine, a horizontal cylindrical mixing machine, a ribbon mixing machine, a high-speed stirring mixing machine, an airflow stirring mixing machine, and the like, or with granulators, for example, such as a fluidized bed granulation drier, a high-speed stirring granulator, and the like.

The masking coating may be formed on the core particle by using, for example, a common pan coating machine, a vented coating machine, a fluidized bed coating device (a fluidized bed granulation and drying device, and the like), a tumbling fluid coating device (a tumbling fluidized bed granulation device, a stirring fluidized bed granulator, and the like), a centrifugal tumbling coating device (a centrifugal tumbling granulation device, and the like), and the like.

The coating liquid may be obtained by dissolving or suspending the constituent components of the masking coating, for example, in an organic solvent such as ethanol, isopropyl alcohol, and acetone, water, a mixed solvent thereof, and the like. However, it is more preferable to dissolve or suspend the constituent components of the masking coating in water to obtain an aqueous coating liquid.

Preferred examples of the coating liquid include one or more liquids selected from (a) organic solvent liquids dissolving at least one polymer selected from methacrylic acid copolymer S, methacrylic acid copolymer L, and ethyl acrylate-methyl methacrylate copolymer, (b) liquids obtained by suspending at least one polymer selected from methacrylic acid copolymer S, methacrylic acid copolymer L, and ethyl acrylate-methyl methacrylate-ethyl ammonium trimethyl chloride methacrylate copolymer in an aqueous solution dissolving 30 to 100 weight% of triethyl citrate with respect to the polymer, (c) liquids obtained by suspending JPE dry methacrylic acid copolymer LD and/or JPE aminoacrylmethacrylate copolymer RS in water, and (d) JPE methacrylic acid copolymer LD and/or JPE ethyl acrylate-methyl methacrylate copolymer dispersion.
More preferred examples of the coating liquid include one or more liquids selected from (c) liquids obtained by suspending JPE dry methacrylic acid copolymer LD and/or JPE aminoacrylmethacrylate copolymer RS in water, and (d) JPE methacrylic acid copolymer LD and/or JPE ethyl acrylate-methyl methacrylate copolymer dispersion.
Further preferred examples of the coating liquid include liquids obtained by suspending JPE dry methacrylic acid copolymer LD in water, and JPE methacrylic acid copolymer LD.

The coating liquid also contains at least one diluent selected from D-mannitol, lactose, trehalose, xylitol, maltitol, and erythritol, preferably at least one diluent selected from D-mannitol and erythritol, more preferably D-mannitol. In the coating liquid, at least one diluent selected from D-mannitol, lactose, trehalose, xylitol, maltitol, and erythritol may be dissolved or suspended, and is preferably dissolved in water. When suspending the diluent in an organic solvent liquid, it is preferable to add an organic solvent to an diluent aqueous solution and precipitate fine crystals to achieve the suspended state.

The present invention also provides an orally-disintegrating tablet that comprises the drug-containing granule of the present invention and a powder for an orally-disintegrating tablet that does not contain a drug causing bitterness (having the same definition as described above).
The powder for an orally-disintegrating tablet of the present invention may be a powder used to produce common orally-disintegrating tablets. Examples include known powders for an orally-disintegrating tablet as described in, for example, WO97/47287, WO2005/004923, Japanese Published Unexamined Patent Application No. 2003-034655, WO2003/074085, and the like. Preferred examples include a powder for an orally-disintegrating tablet that contains a sugar alcohol or a sugar having a volume average particle diameter of 30 µm or less, and a disintegrant (see WO97/47287), a powder for an orally-disintegrating tablet that contains cyclodextrin or a cyclodextrin derivative (see W02005/004923), and the like.

The powder for an orally-disintegrating tablet of the present invention preferably contains sugar and/or sugar alcohol, more preferably at least one substance selected from D-mannitol, lactose, trehalose, xylitol, maltitol, and erythritol. The powder for an orally-disintegrating tablet contains sugar and/or sugar alcohol, preferably at least one selected from D-mannitol, lactose, trehalose, xylitol, maltitol, and erythritol, more preferably at least one selected from D-mannitol, lactose, and erythritol, even more preferably D-mannitol in 50 to 98 weight%, more preferably 60 to 98 weight%, further preferably 70 to 98 weight%.
The sugar and/or sugar alcohol has a volume average particle diameter (having the same definition as described above) of preferably 5 to 150 µm, more preferably 10 to 50 µm, further preferably 15 to 30 µm.

The powder for an orally-disintegrating tablet may contain excipients other than sugar and sugar alcohol. Examples of such excipients include starch, starch derivatives, cellulose, cellulose derivatives, poorly water-soluble inorganic salts (for example, talc, light anhydrous silicic acid, hydrous silicon dioxide, sodium aluminometasilicate, calcium silicate, calcium phosphate, and the like), binders (for example, methylcellulose, carboxymethylcellulose, carboxypropylcellulose, hydroxypropylcellulose, hydroxypropylmethylcellulose, polyvinyl alcohol, polyvinyl pyrrolidone, pullulan, dextrin, gum arabic, gelatin, and the like), disintegrants (for example, crospovidone, bentonite, and the like), lubricants (magnesium stearate, calcium stearate, hardened oil, sucrose fatty acid ester, polyethylene glycol, sodium lauryl sulfate, and the like), sweeteners (for example, saccharine sodium, dipotassium glycyrrhizinate, aspartame, stevia, thaumatin, and the like), acidulants (for example, citric acid, tartaric acid, malic acid, and the like), antioxidizing agents (for example, tocopherol, ascorbic acid, cysteine hydrochloride, L-ascorbyl stearate, and the like), coloring agents (for example, titanium oxide, iron oxide; specifically, yellow ferric oxide, ferric oxide, yellow ferrous oxide, black iron oxide, and the like), zinc oxides, silicon oxides, red iron oxide, carbon blacks, medicinal carbon, barium sulfate, caramel, food yellow 4 aluminum lake, food yellow 5, food red 2, food red 3, food red 102, copper chlorophin, food blue 2, carotenoid dye, tomato dye, and the like), flavoring ingredients (for example, lemon flavor, lemon lime flavor, grapefruit flavor, apple flavor, and the like), and the like. These may be used either alone or in combinations of two or more.
Note that materials such as cellulose (for example, microcrystalline cellulose, powder cellulose, and the like), cellulose derivatives (for example, crosscarmellose sodium, low-substituted hydroxypropylcellulose, carmellose calcium, and the like), starches (for example, corn starch, pregelatinated starch, partially pregelatinated starch, and the like), starch derivatives (hydroxypropyl starch, carboxymethyl starch sodium, and the like), and the like may be contained as disintegrants. These also classify as the disintegrants of the present invention.

Preferred examples of the disintegrant that may be contained in the powder for an orally-disintegrating tablet include crosscarmellose sodium, low-substituted hydroxypropylcellulose, hydroxypropyl starch, carboxymethyl starch sodium, crospovidone, and the like. More preferred examples include crosscarmellose sodium, low-substituted hydroxypropylcellulose, crospovidone, and the like.
Preferably, the powder for an orally-disintegrating tablet contains a disintegrant, preferably at least one disintegrant selected from crosscarmellose sodium, low-substituted hydroxypropylcellulose, hydroxypropyl starch, and crospovidone, more preferably crospovidone in 0.01 to 50 weight%, more preferably 0.1 to 20 weight%, further preferably 1 to 10 weight%. The powder for an orally-disintegrating tablet preferably contains the disintegrant, because it further reduces the disintegration time of the orally-disintegrating tablet in the mouth.

Preferably, the powder for an orally-disintegrating tablet contains a poorly water-soluble inorganic salt, preferably at least one poorly water-soluble inorganic salt selected from talc, light anhydrous silicic acid, hydrous silicon dioxide, sodium aluminometasilicate, calcium silicate, and calcium phosphate, more preferably at least one poorly water-soluble inorganic salt selected from light anhydrous silicic acid, sodium aluminometasilicate, and calcium silicate in 0.01 to 20 weight%, more preferably 0.1 to 10 weight%, further preferably 0.5 to 5 weight%. The powder for an orally-disintegrating tablet preferably contains a poorly water-soluble inorganic salt, because it further reduces the disintegration time of the orally-disintegrating tablet in the mouth. Further, the powder for an orally-disintegrating tablet preferably contains the poorly water-soluble inorganic salt, because it improves the hardness of the orally-disintegrating tablet.

The powder for an orally-disintegrating tablet of the present invention may be a simple mixture with excipients that can be contained in powders for an orally-disintegrating tablet. However, the powder for an orally-disintegrating tablet of the present invention is preferably a granulated powder. When the powder for an orally-disintegrating tablet of the present invention is a granulated powder, the powder is obtained by granulating a powder that contains at least sugar and/or sugar alcohol. When water or binder liquid is used for the granulation, the sugar and/or sugar alcohol, and other excipients that can be contained in the powder for an orally-disintegrating tablet may be added by being partially or entirely dissolved or suspended for granulation.
When the powder for an orally-disintegrating tablet of the present invention is a granulated powder, the powder may be granulated by using methods such as a wet granulation method, and a dry granulation method. Examples of the wet granulation method include an extrusion granulation method (using a screw extrusion granulation device, a roller extrusion granulation device, and the like), a tumbling granulation method (using a rotary drum granulation device, a centrifugal tumbling granulation device, and the like), a fluidized bed granulation method (using a fluidized bed granulation device, a tumbling fluidized bed granulation device, and the like), a stirring granulation method (using a stirring granulation device, and the like), and the like. In any case, it is preferable to, for example, mix excipients that can be contained in the powder for an orally-disintegrating tablet, add a solvent or a binder liquid to the mixture and perform granulation, and dry the resulting granulated material. Examples of the solvent include water, ethanol, isopropyl alcohol, acetone, mixed solvents thereof, and the like. Examples of the binder liquid include solutions obtained by using a binder that can be contained in the powder for an orally-disintegrating tablet, and dissolving the binder in water, ethanol, isopropyl alcohol, acetone, mixed solvents thereof, and the like. The binder liquid is most preferably the solution dissolving the binder in water. The dry granulation method may be, for example, a disintegration granulation method in which flakes are produced with a commercially available dry granulator, or slugs are produced with a tableting machine, and in which the flakes or slugs are crushed with a commercially available crusher or pulverizer to obtain granulated materials, and the like. The granulation is performed more preferably by using a stirring granulation method, a tumbling granulation method, or a fluidized bed granulation method, further preferably by using a stirring granulation method.

The orally-disintegrating tablet of the present invention may be produced by mixing the drug-containing granule of the present invention with a powder for an orally-disintegrating tablet that does not contain a drug causing bitterness (having the same definition as described above), and compression molding the mixture with a tableting machine. When the powder for an orally-disintegrating tablet is a granulated powder, the drug-containing granule of the present invention may be produced by mixing the drug-containing granule of the present invention with the granulated powder for an orally-disintegrating tablet, and, as desired, adding and mixing non-granulated excipients (disintegrant, and the like) that can be contained in the powder for an orally-disintegrating tablet, followed by compression molding with a tableting machine.

In the orally-disintegrating tablet of the present invention, the drug-containing granule preferably contains at least one di luent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer, because it has small effect on the disintegration time of the orally-disintegrating tablet in the mouth (delayed disintegration) and improves the hardness of the orally-disintegrating tablet.

The tableting machine that can be used in the present invention is not particularly limited, and, for example, a rotary tableting machine, a hydraulic press, and the like may be used. It is also possible to use, for example, a tableting machine equipped with punches and dies to which very trace amounts of lubricants such as stearic acid, metal salts thereof (such as magnesium stearate, calcium stearate, and the like), sucrose fatty acid ester, glycerin fatty acid ester, hydrogenated oil and fat, and the like have been applied, and the orally-disintegrating tablet may be produced by so-called external lubrication compression molding.

In the present invention, the orally-disintegrating tablet is preferably, for example, circle-shaped, triangular-shaped, ball-shaped, and the like. The size of the tablet of the present invention is not particularly limited, and is, for example, preferably 0.1 to 1 g in mass, and 0.5 to 1.5 cm in diameter.

In the present invention, the orally-disintegrating tablet preferably has hardness that does not cause, for example, chipping, breaking, and the like. The tablet hardness is generally measured as the break strength of the tablet in diametrical direction, using a tablet hardness meter. The value of tablet harness is preferably 20 to 200 N, more preferably 30 to 150 N, particularly preferably 40 to 100 N. The tablet hardness may be measured by using a commercially available tablet break strength measurement device, for example, a PTB-311E available from Japan Machinery Company.

In the present invention, the disintegration time of the orally-disintegrating tablet in the mouth is preferably 1 minute or less, more preferably 30 seconds or less. For example, the time for a dye solution to completely infiltrate a tablet surface (absorption time) is preferably 1 minute or less, more preferably 30 seconds or less as measured by the absorption time measurement method (see Hisakazu Sunada, Chem. Pharm. Bull, 1996, Vol. 44, No. 11, p. 2121 - 2127), an in vitro test that simulates oral disintegration time.

The present invention is described below more specifically with reference to examples and test examples. It should be noted that the present invention is not limited by the following examples and test examples.

### [Example 1]

2, 000. 0 g of olopatadine hydrochloride (Kyowa Hakko Kirin Co. , Ltd. ; volume average particle diameter of about 30 µm), 9, 120. 0 g of D-mannitol (here and below, Pearlitol 25C; Roquette Japan; volume average particle diameter of about 20 µm), 640.1 g of low-substituted hydroxypropylcellulose (here and below, L-HPC; Shin-Etsu Chemical Co., Ltd.), and 16.0 g of yellow ferric oxide (here and below, Yellow Ferric Oxide; Kishi Kasei) were charged into stirring granulator (here and below, vertical granulator FM-VG-65; Powrex Corporation). These were mixed, and 2, 400. 2 g of methacrylic acid copolymer LD (here and below, Eudragit L30D-55; Roehm Pharma Gmbh) was added for granulation. The resulting granulated material was then wet pulverized with a pulverizer (here and below, Comil QC-194S; Powrex Corporation). The wet pulverized material was dried with a fluidized bed granulation drier (here and below, WSG-30; Powrex Corporation), and dry pulverized with a pulverizer (here and below, power mill P-3; Dalton Co. , Ltd. ) to obtain core particles (weight average particle diameter of about 85 µm).
Separately, 224.0 g of D-mannitol, 112.2 g of triethyl citrate (here and below, Citroflex 2; Morimura Bros., Inc.) and 2, 860. 4 g of water were mixed with one another, and then with 1, 120. 0 g of methacrylic acid copolymer LD and 128.0 g of talc (here and below, Talc; Kihara Kasei) to obtain a coating liquid.
The core particles (1, 000. 1 g) were charged into a fluidized bed granulation drier, and the coating liquid was sprayed to form a 52% coating (by weight) with respect to the core particles. The resulting coated granules (200.0 g) were dried with a constant-temperature isothermal device (here and below, DK 600; Yamato Scientific Co., Ltd.), and sieved with a sieve having 850-µm openings to obtain olopatadine hydrochloride-containing granules (weight average particle diameter of about 250 µm; here and below, weight average particle diameter is measured by sieving).

### [Example 2]

D-mannitol (280.2 g), triethyl citrate (112. 2 g), and water (2, 991. 1 g) were mixed with one another, and then with methacrylic acid copolymer LD (933.4 g) and talc (128. 1 g) to obtain a coating liquid.
The core particles (1, 000. 2 g) obtained in Example 1 were charged into a fluidized bed granulation drier, and the coating liquid was sprayed to form a 52% coating (by weight) with respect to the core particles. The resulting coated granules (200. 0 g) were dried and sieved in the same manner as in Example 1 to obtain olopatadine hydrochloride-containing granules (weight average particle diameter of about 250 µm).

### [Example 3]

D-mannitol (336.0 g), triethyl citrate (112. 1 g), and water (3,121.9 g) were mixed with one another, and then with methacrylic acid copolymer LD (746.8 g) and talc (128.0 g) to obtain a coating liquid.
The core particles (1, 000. 2 g) obtained in Example 1 were charged into a fluidized bed granulation drier, and the coating liquid was sprayed to form a 52% coating (by weight) with respect to the core particles. The resulting coated granules (200. 0 g) were dried and sieved in the same manner as in Example 1 to obtain olopatadine hydrochloride-containing granules (weight average particle diameter of about 250 µm).

### Comparative Example 1

Triethyl citrate (112. 1 g) and water (2, 337. 9 g) were mixed to each other, and then with methacrylic acid copolymer LD (1, 866. 8 g) and talc (128. 0 g) to obtain a coating liquid.
The core particles (1, 000. 2 g) obtained in Example 1 were charged into a fluidized bed granulation drier, and the coating liquid was sprayed to form a 52% coating (by weight) with respect to the core particles. The resulting coated granules (200. 1 g) were dried and sieved in the same manner as in Example 1 to obtain olopatadine hydrochloride-containing granules (weight average particle diameter of about 250 µm).

Table 1

**Table 1 Formulation of olopatadinehydrochloride -containing granule (mg)**

| | content | Example 1 | Example 2 | Example 3 | Comparative Example 1 |
|---|---|---|---|---|---|
| Core particles | olopatadinehydrochloride | 5.0 | | | |
| | D-mannitol | 22.8 | | | |
| | low-substituted hydroxypropylcellulose | 1.6 | | | |
| | methacrylic acid copolymer LD | 1.8 | | | |
| | yellow ferric oxide | 0.04 | | | |
| | total | 31.24 | | | |
| Masking coating | D-mannitol | 4.55 | 5.69 | 6.82 | - |
| | methacrylic acid copolymer LD | 6.82 | 5.69 | 4.55 | 11.37 |
| | talc | 2.60 | 2.60 | 2.60 | 2.60 |
| | triethyl citrate | 2.27 | 2.27 | 2.27 | 2.27 |
| | total | 16.24 | 16.25 | 16.24 | 16.24 |

### Test Example 1

A dissolution test was performed for the olopatadine hydrochloride-containing granules obtained in Examples 1 to 3 and Comparative Example 1 to evaluate bitterness masking.

### "Dissolution Test"

The dissolution test was performed according to JP method 2 (rotation paddle method) under 37°C, 50 rpm conditions using 900 mL of water as the test liquid. A sample solution after 0. 5 min from the start of the testing was quantified for an amount of olopatadine dissolution by high-performance liquid chromatography to evaluate a dissolution profile.

### High-Performance Liquid Chromatography Conditions

Column: Inertsil C8 4.6 x 250 mm; GL Sciences Inc.
Column temperature: constant temperature near 40°C
Mobile phase: 0. 05 mol/L phosphate buffer (pH 3. 5) : acetonitrile = 550 mL: 450 mL + sodium lauryl sulfate 2.3 g
Detection method: UV absorption photometry (wavelength 299 nm)
The results for Test Example 1 are presented in FIG. 1. The olopatadine hydrochloride-containing granules obtained in Examples 1 to 3 and Comparative Example 1 all had dissolution amounts of 30% or less after 0. 5 min, and were able to mask bitterness.

### Test Example 2

A dissolution test was performed for the olopatadine hydrochloride-containing granules obtained in Examples 1 to 3 and Comparative Example 1 to evaluate solubility thereof in the stomach.

### "Dissolution Test"

The dissolution test was performed according to JP method 2 (rotation paddle method) under 37°C, 50 rpm conditions, using 900 mL of JP1 liquid as the test liquid. Sample solutions after 5, 10, 15, and 30 minutes from the start of the testing were quantified for amounts of olopatadine dissolution in the same manner as in Test Example 1 to evaluate a dissolution profile.
The results for Test Example 2 are presented in FIG. 2. In contrast to the olopatadine hydrochloride-containing granules of Comparative Example 1 in which the dissolution amount did not reach 75% in 15 minutes, the olopatadine hydrochloride-containing granules obtained in Examples 1 to 3 had dissolution amounts that exceeded 75% in 15 minutes, demonstrating quick solubility thereof.

### [Example 4]

The core particles (1, 000. 1 g) obtained in Example 1 were charged into a fluidized bed granulation drier, and the coating liquid obtained in Example 1 was sprayed to form a 69% coating (by weight) with respect to the core particles. The resulting coated granules (200. 0 g) were dried and sieved in the same manner as in Example 1 to obtain olopatadine hydrochloride-containing granules (weight average particle diameter of about 250 µm).

### [Example 5]

The core particles (1, 000. 2 g) obtained in Example 1 were charged into a fluidized bed granulation drier, and the coating liquid obtained in Example 2 was sprayed to form a 69% coating (by weight) with respect to the core particles. The resulting coated granules (200. 0 g) were dried and sieved in the same manner as in Example 1 to obtain olopatadine hydrochloride-containing granules (weight average particle diameter of about 250 µm).

### [Example 6]

The core particles (1, 000. 2 g) obtained in Example 1 were charged into a fluidized bed granulation drier, and the coating liquid obtained in Example 3 was sprayed to form a 69% coating (by weight) with respect to the core particles. The resulting coated granules (200. 0 g) were dried and sieved in the same manner as in Example 1 to obtain olopatadine hydrochloride-containing granules (weight average particle diameter of about 250 µm).

### Comparative Example 2

The core particles (1, 000. 2 g) obtained in Example 1 were charged into a fluidized bed granulation drier, and the coating liquid obtained in Comparative Example 1 was sprayed to form a 69% coating (by weight) with respect to the core particles. The resulting coated granules (200. 2 g) were dried and sieved in the same manner as in Example 1 to obtain olopatadine hydrochloride-containing granules (weight average particle diameter of about 250 µm).

Table 2

**Table 2 Formulation of olopatadinehydrochloride -containing granule (mg)**

| | content | Example 4 | Example 5 | Example 6 | Comparative Example 2 |
|---|---|---|---|---|---|
| Core particles | olopatadinehydrochloride | 5.0 | | | |
| | D-mannitol | 22.8 | | | |
| | low-substituted hydroxypropylcellulose | 1.6 | | | |
| | methacrylic acid copolymer LD | 1.8 | | | |
| | yellow ferric oxide | 0.04 | | | |
| | total | 31.24 | | | |
| Masking coating | I)-mannitol | 6.04 | 7.55 | 9.05 | - |
| | methacrylic acid copolymer LD | 9.06 | 7.55 | 6.04 | 15.09 |
| | talc | 3.45 | 3.45 | 3.45 | 3.45 |
| | triethyl citrate | 3.01 | 3.01 | 3.01 | 3.01 |
| | total | 21.55 | 21.56 | 21.55 | 21.55 |

### Test Example 3

A dissolution test was performed for the olopatadine hydrochloride-containing granules obtained in Examples 4 to 6 and Comparative Example 2 to evaluate bitterness masking.

### "Dissolution Test"

The dissolution test was performed in the same manner as in Test Example 1. A sample solution after 0.5 minutes from the start of the testing was quantified for an amount of olopatadine dissolution in the same manner as in Test Example 1 to evaluate a dissolution profile.
The results for Test Example 3 are presented in FIG. 3. The olopatadine hydrochloride-containing granules obtained in Examples 4 to 6 and Comparative Example 2 all had dissolution amounts of 30% or less after 0. 5 minutes, and were able to mask bitterness.

### Test Example 4

A dissolution test was performed for the olopatadine hydrochloride-containing granules obtained in Examples 4 to 6 and Comparative Example 2 to evaluate solubility thereof in the stomach.

### "Dissolution Test"

The dissolution test was performed in the same manner as in Test Example 2. Sample solutions after 5, 10, 15, and 30 minutes from the start of the testing were quantified for
amounts of olopatadine dissolution in the same manner as in Test Example 1 to evaluate a dissolution profile.
The results for Test Example 4 are presented in FIG. 4. In contrast to the olopatadine hydrochloride-containing granules of Comparative Example 2 in which the dissolution amount did not reach 75% in 15 minutes, the olopatadine hydrochloride-containing granules obtained in Examples 4 to 6 had dissolution amounts that exceeded 75% in 15 minutes, demonstrating quick solubility thereof.

### [Example 7]

Olopatadine hydrochloride (920. 0 g), D-mannitol (4, 195. 2 g), low-substituted hydroxypropylcellulose (294. 4 g), and yellow ferric oxide (7. 36 g) were charged into a stirring granulator. These were mixed, and methacrylic acid copolymer LD (1, 104. 1 g) was added for granulation. The granulation step was repeated twice. The resulting granulated material was wet pulverized with a pulverizer, dried with a fluidized bed granulation drier, and dry pulverized with a pulverizer to obtain core particles (weight average particle diameter of about 125 µm).
Separately, D-mannitol (1,728.0 g), triethyl citrate (192. 1 g), and water (11, 791. 0 g) were mixed with one another, and then with methacrylic acid copolymer LD (5, 760. 0 g) and talc (640. 0 g) to obtain a coating liquid.
The resulting core particles (9,996.8 g) were charged into a fluidized bed granulation drier, and the coating liquid (29, 111. 0 g) was sprayed to form a masking coating. The resulting coated granules were sieved with a sieve having 710-µm openings to obtain olopatadine, hydrochloride-containing granules (weight average particle diameter of about 200 µm).

Table 3

**Table 3 Formulation of olopatadinehydrochloride -containing granule (mg)**

| content | (mg) |
|---|---|
| olopatadinehydrochloride | 5.0 |
| D-mannitol (Core particles) | 22.8 |
| low-substituted hydroxypropylcellulose | 1.6 |
| methacrylic acid copolymer LD(Core particles) | 1.8 |
| yellow ferric oxide | 0.04 |
| D-mannitol(Masking coating) | 5.4 |
| methacrylic acid copolymer LD(Masking coating) | 5.4 |
| talc | 2.0 |
| triethyl citrate | 0.6 |
| total | 44.64 |

### [Example 8]

4, 815. 4 g of D-mannitol, 234. 7 g of crospovidone (here and below, PVPP XL-10; ISP Japan), 23.5g of light anhydrous silicic acid (here and below, Adsolider 101, Y. K. F. ) (this and other products are actively listed), and 4. 7 g of yellow ferric oxide were charged into a vertical granulator (FM-VG-25P, Powrex Corporation). These were mixed, and 1, 223. 3 g of water was added for granulation. The resulting granulated material was wet pulverized with a pulverizer. The wet pulverized material was dried with a fluidized bed granulation drier, and dry pulverized with a pulverizer. This was repeated several times to obtain a powder for an orally-disintegrating tablet.
The resulting powder for an orally-disintegrating tablet (27, 801. 6 g) was mixed with the olopatadine hydrochloride-containing granules (7, 142. 4 g) obtained in Example 7, 33. 6 g of aspartame (here and below, aspartame; Ajinomoto Co., Inc. ), a flavoring ingredient (33. 6 g), and 64. 0 g of magnesium stearate (here and below, Magnesium Stearate; Taihei Chemical Industrial Co. , Ltd.) using a mixing machine (here and below, twin blade mixer TBM-150; Tokuju Co., Ltd. ) to obtain tableting powder particles
The tableting powder particles were punched using a tableting machine (HT-AP15SS, Hata Iron Works, Co. , Ltd. ) equipped with an external unit that blows the powder particles to the punch and die in amounts that make the magnesium stearate about 0.4 mg per tablet. As a result, olopatadine hydrochloride-containing orally-disintegrating tablets (radial direction tablet hardness 62 N; PTB-311E available from Japan Machinery Company was used) were obtained.

Table 4

**Table 4 Formulation of olopatadinehydrochloride -containing orally-disintegrating tablet (mg) )**

| content | (mg) |
|---|---|
| olopatadinehydrochloride | 5.0 |
| D-mannitol | 193.0 |
| low-substituted hydroxypropylcellulose | 1.6 |
| crospovidone | 8.0 |
| light anhydrous silicic acid | 0.8 |
| methacrylic acid copolymer LD | 7.2 |
| talc | 2.0 |
| triethyl citrate | 0.6 |
| aspartame | 0.21 |
| flavoring ingredient | 0.21 |
| yellow ferric oxide | 0.20 |
| magnesium stearate | 0.8 |
| total | 219.62 |

### Test Example 5

A bitterness sensory test was conducted for the olopatadine hydrochloride-containing orally-disintegrating tablets obtained in Example 8.

### "Bitterness Sensory Testing Method"

One of the olopatadine hydrochloride-containing orally-disintegrating tablets was put in the mouth, and kept in the mouth until the tablet disintegrated. The test sample was removed from the mouth, and bitterness was evaluated after rinsing the mouth with water. Evaluation was made according to the following criteria scores.

### Criteria Scores

5: No bitterness
4: Bitterness is present, but mild enough to be ignored
3: Bitterness is present, but the tablet is ingestible considering that it is a drug
2: Harsh bitterness, taking the tablet involves effort
1: Too bitter to swallow
The average score of the testing participated by seven adults was 4. 4, demonstrating that the olopatadine hydrochloride-containing orally-disintegrating tablets obtained in Example 8 had the sufficient ability to mask bitterness.

### Test Example 6

The olopatadine hydrochloride-containing orally-disintegrating tablets obtained in Example 8 were evaluated for bitterness masking by a syringe barrel inversion method.
A single tablet was inserted into a syringe barrel, and, after adding water (5 mL), the content was stirred for a predetermined time period by rotating the barrel once in every 10 seconds. A sample solution filtered through a membrane filter (10, 20, and 30 seconds) was then quantified for an amount of olopatadine dissolution by high-performance liquid chromatography in the same manner as in Test Example 1 to evaluate a dissolution profile. The result for Test Example 6 is presented in FIG. 5.
The amount of dissolution after 30 seconds was 30% or less, demonstrating that bitterness was masked.

### Test Example 7

The olopatadine hydrochloride-containing orally-disintegrating tablets obtained in Example 8 were evaluated for disintegrability by absorption time measurement.

### "Absorption Time Measurement"

An aqueous solution (2 mL) of 10 mg/mL yellow 5 was dropped onto a circular filter paper having a diameter of 55 mm. One of the olopatadine hydrochloride-containing orally-disintegrating tablets obtained in Example 8 was then placed on the wetted filter paper, and the time required for the dye solution to completely infiltrate the tablet surface was measured. The mean value of absorption times from two measurements was 17 seconds, demonstrating that the tablet disintegrates quickly.

### Test Example 8

A dissolution test was performed for the olopatadine hydrochloride-containing orally-disintegrating tablets obtained in Example 8 to evaluate solubility thereof in the stomach.

### "Dissolution Test"

The dissolution test was performed in the same manner as in Test Example 2. Sample solutions after 5, 10, 15, and 30 minutes from the start of the testing were quantified for amounts of olopatadine dissolution in the same manner as in Test Example 1 to evaluate a dissolution profile.
The result for Test Example 7 is presented in FIG. 6. The olopatadine hydrochloride-containing orally-disintegrating tablets obtained in Example 8 had dissolution amounts that exceeded 75% in 15 minutes, demonstrating quick solubility thereof.

### [Industrial Applicability]

The present invention can provide a powder, a granule, an orally-disintegrating tablet, and the like that contain a drug causing bitterness, and that can suppress the bitterness in the mouth (suppress an amount of dissolution thereof in the mouth) and improve solubility thereof in the stomach.

## Claims

1. A drug-containing granule comprising (a) a core particle that contains a drug causing bitterness, and (b) a masking coating that coats the core particle,
wherein the masking coating contains:
at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer in 20 to 70 weight% of the coating; and
at least one diluent selected from D-mannitol, lactose, trehalose, xylitol, maltitol, and erythritol in 40 to 250 weight% of the polymer.

2. A drug-containing granule comprising (a) a core particle that contains cinacalcet, topiramate, olopatadine, or a pharmaceutically acceptable salt thereof as a drug causing bitterness, and (b) a masking coating that coats the core particle,
wherein the masking coating contains methacrylic acid-ethyl acrylate copolymer, and at least one diluent selected from D-mannitol, lactose, trehalose, xylitol, maltitol, and erythritol.

3. The drug-containing granule according to Claim 1 or 2, wherein the core particle contains at least one diluent selected from D-mannitol, lactose, trehalose, xylitol, maltitol, and erythritol, and at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.

4. The drug-containing granule according to any one of Claims 1 to 3, wherein the drug-containing granule has a weight average particle diameter of 100 to 350 µm.

5. An orally-disintegrating tablet comprising the drug-containing granule of any one of Claims 1 to 4, and a powder for an orally-disintegrating tablet that does not contain the drug causing bitterness.

6. A process for producing a drug-containing granule that comprises (a) a core particle that contains a drug causing bitterness, and (b) a masking coating that coats the core particle,
the process comprising the steps of:
producing the core particle; and
forming a masking coating by coating the core particle with a coating liquid that contains:
at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer in 20 to 70 weight% of the masking coating; and
at least one di luent selected from D-mannitol, lactose, trehalose, xylitol, maltitol, and erythritol in 40 to 250 weight% of the polymer.

7. The process according to Claim 6, wherein the step of producing the core particle that contains a drug causing bitterness is the step of granulating the drug causing bitterness and at least one diluent selected from D-mannitol, lactose, trehalose, xylitol, maltitol, and erythritol with a binder liquid that contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.

8. A process for producing a drug-containing granule that comprises (a) a core particle that contains cinacalcet, topiramate, olopatadine, or a pharmaceutically acceptable salt thereof as a drug causing bitterness, and (b) a masking coating that coats the core particle,
the process comprising the steps of:
producing the core particle; and
forming a masking coating by coating the core particle with a coating liquid that contains methacrylic acid-ethyl acrylate copolymer, and at least one diluent selected from D-mannitol, lactose, trehalose, xylitol, maltitol, and erythritol.

9. The process according to Claim 8, wherein the step of producing the core particle is the step of granulating cinacalcet, topiramate, olopatadine, or a pharmaceutically acceptable salt thereof, and at least one diluent selected from D-mannitol, lactose, trehalose, xylitol, maltitol, and erythritol with a binder liquid that contains at least one polymer selected from methacrylic acid copolymer S, methacrylic acid copolymer L, methacrylic acid-ethyl acrylate copolymer, ethyl acrylate-methyl methacrylate copolymer, and ethyl acrylate-methyl methacrylate-ethyl ammonium trimethyl chloride methacrylate copolymer.

10. The process according to any one of Claims 6 to 9, wherein the core particle is made to have a weight average particle diameter of 75 to 150 µm.

11. A process for producing an orally-disintegrating tablet,
the process comprising the steps of:
mixing the drug-containing granule obtained by the process of any one of Claims 6 to 10 with a powder for an orally-disintegrating tablet that does not contain the drug causing bitterness; and
compressing the mixed powder.
